(19) Europäisches Patentamt
European Patent Office
Office européen des brevets

(11) Veröffentlichungsnummer: **0 184 150**
**B1**

(12) # EUROPÄISCHE PATENTSCHRIFT

(45) Veröffentlichungstag der Patentschrift: **23.05.90**

(51) Int. Cl.⁵: **C07C 29/17**, C07C 33/025

(21) Anmeldenummer: **85115153.0**

(22) Anmeldetag: **29.11.85**

(54) **Verfahren zur Herstellung von olefinisch ungesättigten Verbindungen, insbesondere Alkenolen.**

(30) Priorität: **04.12.84 DE 3444112**

(43) Veröffentlichungstag der Anmeldung: **11.06.86 Patentblatt 86/24**

(45) Bekanntmachung des Hinweises auf die Patenterteilung: **23.05.90 Patentblatt 90/21**

(84) Benannte Vertragsstaaten: **AT BE CH DE FR GB IT LI NL**

(56) Entgegenhaltungen:
**EP-A- 0 011 439**
**EP-A- 0 032 396**
**EP-A- 0 044 413**
**FR-A- 2 276 873**

(73) Patentinhaber: **BASF Aktiengesellschaft, Carl-Bosch-Strasse 38, D-6700 Ludwigshafen(DE)**

(72) Erfinder: **Seufert, Walter, Dr., Laerchenweg 19, D-6720 Speyer(DE)**
Erfinder: **Goetz, Norbert, Dr., Schoefferstrasse 25, D-6520 Worms 1(DE)**
Erfinder: **Becker, Rainer, Dr., Im Haseneck 22, D-6702 Bad Duerkheim(DE)**
Erfinder: **Schwendemann, Volker, Dr., 35 Oak Lane, Mountain Lakes, N.J. 07046(US)**

**EP 0 184 150 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung der cis-Isomeren von Alkenylverbindungen der Formel I

$$CH_3(CH_2)_m\text{–}CH\text{=}CH\text{–}(CH_2)_n\text{–}OR \quad (I)$$

in der R Wasserstoff oder eine leicht abspaltbare Schutzgruppe, z.B. einen Ether- oder Esterrest, m die Werte 1 bis 12 und n die Werte 1 bis 10 bedeuten, durch partielle Hydrierung der entsprechenden Alkinylverbindungen an einem vorhydrierten Trägerkatalysator, der Palladium enthält.

Für viele, insbesondere biologische Zwecke, z.B. bei Geruchsstoffen, Wachstumsregulatoren, Pharmaka, kommt es auf die Reinheit, und zwar, wo dies strukturell gegeben ist, auch auf die sterische Einheitlichkeit der eingesetzten Verbindungen an. Weiterhin ist wichtig, daß diese Stoffe in leicht beschaffbaren, z.B. in Labor- oder Technikumsapparaten aus Glas hergestellt werden können, d.h. daß man auf die Anwendung hohen Drucks verzichten kann.

An sich ist bekannt, daß die Anlagerung von Wasserstoff an die C≡C-Dreifachbindung unter Bildung der cis-Olefine verlaufen kann (vgl. Houben-Weyl, Methoden der Organischen Chemie, 4. Aufl., Band 4/1c, Seiten 76 ff und 110 ff). Dabei erweist sich der sogenannte Lindlar-Katalysator (Palladium-Katalysator auf Kalziumcarbonat-Basis mit Bleiacetet-Zusatz) in der Regel in der sterischen Einheitlichkeit der Hydrierprodukte anderen Katalysatoren überlegen und ist auch schon bei atmosphärischem Druck wirksam. Aber auch mit dem Lindlar-Katalysator entstehen neben dem erwünschten cis-Isomeren erhebliche Mengen des Trans-Isomeren; erfahrungsgemäß liegt das cis/trans-Verhältnis in der Nähe von 10:1 oder ungünstiger.

Hohe Zahlenwerte der sterischen Einheitlichkeit scheinen zwar bei der in EP-A 11 439 beschriebenen Methode erzielbar; jedoch erfordert die vorgeschlagene Hydrierung mindestens 5 bar Wasserstoffdruck im Autoklaven, also den Einsatz teurer Spezialapparaturen, und es wird ein Katalysator mit hohem Edelmetallgehalt, nämlich 3 bis 15% Palladium benötigt. Der Katalysator kann 0,5 bis 10% Zink enthalten.

Aus der EP-A 44 413 ist bekannt, Verbindungen mit endständiger olefinischer Doppelbindung durch Hydrierung herzustellen, wobei Palladium-Trägerkatalysatoren verwendet werden, deren Träger Calciumcarbonat ist und die Zink enthalten. Die Aufgabe, cis-Isomere von Olefinen herzustellen, ist jedoch dort nicht angesprochen. Dies gilt auch für die Angaben, die der FR-A 2 276 873 entnommen werden können.

Es bestand daher ein Bedürfnis nach einem technisch einsetzbaren Verfahren, das es erlaubt, acetylenisch ungesättigte Verbindungen mit innenständiger Dreifachbindung, insbesondere längerkettige Alkinole bzw. deren Derivate möglichst vollständig und nebenproduktarm zu den cis-Alkenolen zu hydrieren. Die acetylenisch ungesättigten Verbindungen sind oft besonders leicht und in guter Reinheit erhältlich.

Die Lösung der Aufgabe besteht in dem Verfahren nach den Patentansprüchen. Es ist überraschend, daß ein mit Zink oder Cadmium abgeschwächter Palladiumkatalysator praktisch bei atmosphärischem Druck sehr selektiv Dreifachbindungen zu olefinischen Doppelbindungen zu hydrieren vermag, wobei die Hydrierung bei Erreichen des olefinischen Zustandes zum Stillstand kommt, d.h. die Wasserstoffmenge nicht begrenzt werden muß.

Der erfindungsgemäß zu verwendende Katalysator enthält Palladium und Zink und/oder Cadmium auf einem Caloiumcarbonatträger. Der Palladium-Gehalt, bezogen auf den Katalysator einschließlich Trägermaterial, liegt im Bereich von $5 \times 10^{-2}$ bis 5,
vorzugsweise $10^{-1}$ bis 3 Gew.%, berechnet als Metall, der des Zinks bzw. Cadmiums $5 \times 10^{-2}$ bis 15, vorzugsweise $10^{-1}$ bis 10 Gew.%. Das Gewichtsverhältnis von Zink und/oder Cadmium zu Palladium kann unterschiedlich sein, z.B. im Bereich von 100:1 bis 1:300. Günstig sind Katalysatoren, die relativ wenig Palladium (z.B. 0,1 bis 2%) und eine jeweils etwas höhere Menge Zink bzw. Cadmium enthalten. Die Herstellung geeigneter Palladium/Zink/Cadmium-Katalysatoren ist z.B. in der EP-A 50 229 oder der DE-A 3 039 085 beschrieben.

Man verwendet den Katalysator meist in Form von Strängen (beim Hydrieren im Festbett) oder als Pulver (beim Arbeiten in Suspension).

Geeignete Lösungsmittel sind vor allem Alkohole, insbesondere $C_1$- bis $C_4$-Alkohole und Gemische von solchen mit Wasser; auch reines Wasser ist geeignet, obwohl die beteiligten Verbindungen darin i.a. nur wenig löslich sind.

Die Reaktion verläuft schon oberhalb von etwa –10°C befriedigend rasch, vorzugsweise etwas unterhalb von Raumtemperatur. Der erforderliche Wasserstoffmindestdruck in der Regel von atmosphärischem Druck bis etwa 5 bar, vorzugsweise bis 2 bar; ein Druck von 5 bar oder mehr, z.B. bis 10 bar ist zwar möglich, aber im allgemeinen unwirtschaftlich und erfordert besondere Apparate, da die Umsetzung dann sehr rasch verläuft; u.U. tritt bei zu hohem Druck auch der gewünschte Erfolg nicht ein. Die Selektivität des Katalysators kann weiter erhöht werden, wenn bei der Reaktion ein (tertiäres oder quasi-aromatisches) Amin zugegen ist; geeignet sind z.B. Morpholine, Chinoline, niedere Alkylamine, Pyridin; bewährt hat sich z.B. 4-Methylmorpholin, also ein tertiäres Amin, das leicht wieder abgetrennt werden kann, in einer Menge, die i.a. 15 Gew.%, bezogen auf den Ausgangsstoff, nicht zu übersteigen braucht.

R in Formel I ist vorzugsweise Wasserstoff. Als leicht abspaltbare Schutzgruppe, die den Wasserstoff ersetzen kann, kommen beispielsweise in Betracht: Tetrahydropyranyl-2, Trialkylsilyl, Acetyl, tert.-Butyl und Benzyl. Natürlich können auch Verbindungen in die cis-Olefinstruktur überführt werden, bei denen R keine Schutzgruppe bedeutet, sondern einen Bestandteil des Endprodukts, wie z.B. Ester der Hydroxylverbindungen mit gesättigten oder ungesättigten Fettsäuren oder Ether, vorzugsweise Alkylether.

Beispiel 1

6212 g (34,8 Mol) Dodecin-9-ol-1 und 316 g N-Methylmorpholin werden in 46 l Methanol in einem 100 l-Glaskolben gelöst und 62 g Katalysator (0,7 % Pd und 3,1 % Zn auf $CaCO_3$-Basis) in der Lösung aufgeschlämmt. Dann wird unter Rühren mit Stickstoff gespült und bei 0 bis 5°C unter 0,1 bar Wasserstoffüberdruck bis zum Ende der Wasserstoffaufnahme hydriert. Nach Abziehen des Methanols und Morpholins erhält man 6200 g Dodecen-9-ol-1 mit einem cis-Anteil von 96,6 % und einem trans-Anteil von 2,1 %.

Nach der vorstehenden, entsprechend angepaßten Vorschrift wurden folgende Ergebnisse (Tabelle) erhalten.

Tabelle 1

| Beispiel | Alkenol | cis-Anteil (%) | trans-Anteil (%) |
|---|---|---|---|
| 2 | Tetradecen-9-ol-1 | 97,4 | 2,1 |
| 3 | Tetradecen-11-ol-1 | 96,2 | 3,0 |
| 4 | Hexadecen-11-ol-1 | 96,7 | 2,5 |
| 5 | Hexadecen-7-ol-1 | 98,6 | 1,4 |
| 6 | Tetradecen-5-ol-1 | 95,4 | 2,6 |
| 7 | Tetradecen-7-ol-1 | 96,0 | 1,8 |

Beispiel 8 bis 15

Es wurden jeweils 40 g Dodecin-9-ol-1 in Gegenwart von 1,2 g N-Methylmorpholin in 170 ml Methanol hydriert, wobei Katalysatorart und -menge variiert wurden. Bei diesen Beispielen ist zu beachten, daß durch die besonders geringen Mengen die Steuerbarkeit der Umsetzung erschwert ist. Die Beispiele sind daher nur untereinander, nicht aber mit den vorstehenden Beispielen vergleichbar (vgl. B. 1 mit 8l).

Tabelle 2

| Beispiel | Katalysator Gew.%/Träger $CaCO_3$ | Katalysatormenge (g) | cis-Anteil (%) | trans-Anteil (%) |
|---|---|---|---|---|
| 8 | 0,7 Pd, 3,2 Zn | 0,4 | 94,0 | 2,9 |
| 9 | 0,7 Pd, 1,0 Zn. | 0,4 | 92,7 | 4,4 |
| 10 | 0,3 Pd, 3,2 Zn | 0,4 | 88,8 | 5,9 |
| 11 | 2,9 Pd, 3,2 Zn | 0,4 | 92,7 | 5,0 |
| 12 | 2,9 Pd, 3,2 Zn | 0,05 | 87,6 | 6,2 |
| 13 | 0,7 Pd, 6,3 Zn | 0,4 | 95,5 | 4,2 |
| 14 | 0,7 Pd, 3,1 Zn/3,1 Cd | 0,4 | 95,0 | 3,2 |
| 15 | 0,7 Pd, 5,4 Cd | 0,4 | 94,0 | 3,7 |

Beispiele 16 und 17

Die Umsetzung wird, wie in Beispiel 1 beschrieben, in wasserhaltigem Methanol vorgenommen. Bei einem Wassergehalt von 1 % wurde ein cis/trans-Verhältnis von 95,6 zu 3,1, bei 5 % ein cis/trans-Verhältnis von 93,6 zu 4,2 beobachtet.

## Patentansprüche

1. Verfahren zur Herstellung des cis-Isomeren einer Alkenylverbindung der Formel

$$CH_3(CH_2)_m\text{–}CH\text{=}CH\text{–}(CH_2)_n\text{–}OR \qquad (I),$$

wobei R Wasserstoff, einen Ether- oder Esterrest oder eine leichtabspaltbare Schutzgruppe bedeutet, m die Werte 1 bis 12 und n die Werte 1 bis 10 annehmen kann, durch Hydrierung von entsprechenden acetylenisch ungesättigten Verbindungen an einem vorhydrierten Palladiumkatalysator, dadurch gekennzeichnet, daß man einen Trägerkatalysator verwendet, der Calciumcarbonat als Träger und 0,05 bis 5 Gew.% Palladium und 0,05 bis 15 Gew.% Zink oder Cadmium enthält.

2. Verfahren nach Anspruch 1, dadurch gekennzeichnet, daß der Katalysator 3 Gew.% oder weniger an Palladium enthält.

3. Verwendung des Verfahrens nach Anspruch 1 oder 2 zur Herstellung des cis-Isomeren einer entsprechenden Alkenylhydroxiverbindung der Formel

$$CH_3(CH_2)_m\text{–}CH\text{=}CH\text{–}(CH_2)_nOH \qquad (II).$$

4. Verfahren nach Anspruch 3 zur Herstellung von Z-9-Dodecenol, Z-9-Tetradecenol, Z-11-Tetradecenol oder Z-11-Hexadecenol.

5. Verfahren nach einem der Ansprüche 1 bis 4, dadurch gekennzeichnet, daß man die Umsetzung in Gegenwart eines tertiären Amins oder eines quasi-aromatischen Amins vornimmt.

6. Verfahren nach Anspruch 5, dadurch gekennzeichnet, daß man ein N-Alkylmorpholin verwendet.

## Claims

1. A process for the preparation of the cis-isomer of an alkenyl compound of the formula

$$CH_3(CH_2)_m\text{–}CH\text{=}CH\text{–}(CH_2)_n\text{–}OR \qquad (I)$$

where R is hydrogen, an ether or ester radical, or a protective group which can readily be eliminated, m is from 1 to 12 and n is from 1 to 10, by hydrogenating the correspondig acetylenically unsaturated compound over a palladium catalyst, wherein a supported catalyst which contains calcium carbonate as the carrier and from 0.05 to 5% by weight of palladium and from 0.05 to 15% by weight of zinc or cadmium is used.

2. A process as claimed in claim 1, wherein the catalyst contains 3% by weight of palladium or less.

3. Use of a process as claimed in claim 1 or 2 for the preparation of the cis-isomer of a corresponding alkenylhydroxy compound of the formula

$$CH_3(CH_2)_m\text{–}CH\text{=}CH\text{–}(CH_2)_nOH \qquad (II).$$

4. A process as claimed in claim 3 for the preparation of Z-9-dodecenol, Z-9-tetradecenol, Z-11-tetradecenol or Z-11-hexadecenol.

5. A process as claimed in claim 1 or 2 or 3 or 4, wherein the reaction is carried out in the presence of a tertiary amine or of a quasi-aromatic amine.

6. A process as claimed in claim 5, wherein an N-alkylmorpholine is used.

## Revendications

1. Procédé de préparation de l'isomère-cis d'un dérivé d'alcényle de formule

$$CH_3(CH_2)_m\text{–}CH\text{=}CH\text{–}(CH_2)_n\text{–}OR \qquad (I),$$

dans laquelle R représente hydrogène, un reste éther ou ester ou un groupe protecteur facilement éliminable, m, les valeurs 1 à 12 et n, les valeurs 1 à 10, par hydrogénation de composés correspondants à in-

saturation acéthylénique sur un catalyseur au palladium, caractérisé par le fait que l'on utilise un catalyseur qui contient du carbonate de calcium comme support et 0,05 à 5% en poids de palladium et 0,05 à 15% en poids de zinc ou de cadmium.

2. Procédé selon la revendication 1, caractérisé par le fait que le catalyseur contient 3% en poids ou moins de palladium.

3. Utilisation du procédé selon les revendications 1 ou 2 pour préparer l'isomère-cis d'un dérivé alcénylhydroxylé correspondant, de formule

$$CH_3(CH_2)_m-CH=CH-(CH_2)_nOH \qquad (II).$$

4. Procédé selon la revendication 3 pour la préparation de Z-9-dodécénol, Z-9-tétradécénol, Z-11-tétradécénol ou Z-11-hexadécénol.

5. Procédé selon l'une des revendications 1 à 4, caractérisé par le fait que l'on effectue la réaction en présence d'une amine tertiaire ou d'une amine quasi aromatique.

6. Procédé selon la revendication 5, caractérisé par le fait que l'on utilise une N-alkylmorpholine.